# EUROPÄISCHE PATENTSCHRIFT

(11) **EP 1 633 152 B1**
(45) Veröffentlichungstag und Bekanntmachung des Hinweises auf die Patenterteilung: **25.08.2010**
(21) Anmeldenummer: 05018976.0
(22) Anmeldetag: 01.09.2005
(51) Int. Cl.: A61N 1/372

(54) **Verfahren zum Betrieb eines Kommunikationsmoduls**
Method for the operation of a communications module
Procédé d'exploitation d'un module de communication

(30) Priorität: 03.09.2004 DE 102004043212
(43) Veröffentlichungstag der Anmeldung: 08.03.2006
(73) Patentinhaber: Biotronik CRM Patent AG, 6340 Baar/Zug (CH)
(72) Erfinder: Elsner, Joachim, Dr., 14059 Berlin (DE); Gromotka, Bernhard, 12359 Berlin (DE); Lang, Martin, Dr., 91085 Weisendorf (DE); Merlin, Julian, 10713 Berlin (DE)
(74) Vertreter: Lindner-Vogt, Karin L.

(56) Entgegenhaltungen:
- WO-A-03/008013
- DE-A1- 19 600 922
- DE-A1- 19 930 263
- DE-A1- 19 959 545
- US-A1- 2003 114 898

## Beschreibung

Die vorliegende Erfindung betrifft ein Verfahren zur zeitlichen Abstimmung der Aktivitätsphasen eines Patientengerätes und eines elektromedizinischen Implantates als Kommunikationspartner.

Kommunikationspartner, die jeweils nur kurzzeitig sendend und empfangend miteinander kommunizieren, und die Verfahren sie zu betreiben sind im Stand der Technik insbesondere dort bekannt, wo eine geringe zur Verfügung stehende Sendebandweite auf eine Mehrzahl von Sendern und Empfängern verteilt werden muss. Da nicht beliebig viel Sendebandweite zur Verfügung steht, müssen die Kommunikationsteilnehmer effizient damit umgehen. So schreiben nationale und internationale Standards für bestimmte freigegebene Frequenzbänder das sogenannte Listen-before-Transmit-Verfahren (LBT) vor, demzufolge jeder Kommunikationsteilnehmer, der in dem besagten Frequenzband senden will, zuerst den vorhandenen Sendeverkehr beobachten muss, bevor er nach einer vorgeschriebenen Zeitspanne, in der es zu keinem Funkverkehr in dem Frequenzband gekommen ist, selber funken darf. Gewöhnlich verbleibt dabei ein Kommunikationsteilnehmer im Empfangsmodus und wartet auf ein Erkennungssignal des Kommunikationspartners, das die Bereitschaft zur Aufnahme einer wechselseitigen Kommunikation signalisiert.

Moderne elektromedizinische Implantate, insbesondere Herzschrittmacher, Defibrillatoren und dergleichen, bieten Arzt und Patienten ein Höchstmaß an Sicherheit und Komfort durch so genannte Home Monitoring Funktionen.

Dabei protokolliert das Implantat Diagnose- und Therapieinformationen und überträgt diese Informationen über eine Telemetrieschnittstelle an ein tragbares externes Patientengerät. Von dort werden die Daten an das Home Monitoring Service Center weitergeleitet, wo sie für den Arzt gespeichert und visualisiert werden. Der Arzt kann sich auf diese Weise direkt über den Therapieverlauf und den aktuellen Gesundheitszustand seiner Patienten informieren. Er hat damit die Möglichkeit, schnell auf Veränderungen des Gesundheitszustandes seiner Patienten zu reagieren.

Ohne Home Monitoring kann der Arzt diese Informationen nur im Rahmen einer Untersuchung des Patienten abfragen. In kritischen Situationen führt dies zu unerwünschten Verzögerungen im Informationsfluss. Zudem ist jede Untersuchung für Arzt und Patienten mit erheblichem Zeitaufwand verbunden. Häufige Untersuchungen führen insbesondere für den Patienten zu einer Einschränkung von Mobilität und Lebensqualität.

Mit Home Monitoring werden die Implantatinformationen über die beschriebene technische Vorrichtung (siehe auch US 6.553.262, US 5.752.976) im Hintergrund verschickt, ohne dass der Patient in der normalen Lebensführung eingeschränkt wird; d.h., er hat die Sicherheit des ärztlichen Home Monitorings ohne die Belastung durch häufige Untersuchungen.

Die mittelbare Kommunikation zwischen Implantat und Home Monitoring Service Center ist deshalb vonnöten, weil das Implantat wiederum aufgrund der strengen Anforderungen an die Leistungsaufnahme nur auf kurze Distanz funken kann. Durch den geringen Abstand zwischen Patientengerät und elektromedizinischem Implantat ist außerdem gewährleistet, dass das Sendesignal des Patientengerätes trotz der Abschirmung durch den Körper des Patienten stark genug ist, um vom Implantat empfangen zu werden.

Im Fall eines solchen sich im Körper eines Patienten befindenden elektromedizinischen Implantates entsteht jedoch die Schwierigkeit, dass das Implantat durch den umgebenden Körper abgeschirmt wird, so dass schwache Signale, die gerade den Sendekanal belegen, nicht wahrgenommen werden können. Das elektromedizinische Implantat könnte so fälschlicherweise den Sendekanal als unbenutzt ansehen, für sich in Anspruch nehmen und die Kommunikation anderer Geräte stören. Ein LBT-Protokoll kann deshalb nicht von einem elektromedizinischen Implantat initiiert werden, weshalb eine Kommunikation immer durch das externe Patientengerät, welches in der Lage ist, den Sendekanal mit der notwendigen Empfindlichkeit abzuhören, eingeleitet werden muss.

Die ununterbrochene Empfangsbereitschaft des Empfängers bedeutet außerdem für ein batteriebetriebenes Kommunikationsgerät einen unter Umständen unvertretbar hohen zusätzlichen Strombedarf. Dies gilt insbesondere für elektromedizinische Implantate wie zum Beispiel einen Herzschrittmacher, die sich über mehrere Jahre hinweg betriebsbereit im Körper eines Patienten befinden, ohne dass die Möglichkeit zum Aufladen oder Austauschen der Batterien gegeben wäre. In dem Fall der elektromedizinischen Implantate wäre es daher vorteilhaft, auch den Empfänger fortlaufend abschalten zu können und ihn nur zu den Zeitpunkten des erwarteten Sendens seitens des Kommunikationspartners wieder anzuschalten.

Um dem Patienten eine möglichst uneingeschränkte Bewegungsfreiheit zu gewähren, kommuniziert das Patientengerät als tragbare Ausführung mit dem Home Monitoring Service Center gewöhnlich über eine drahtlose Daten- oder Telefonverbindung. Da an zahlreichen Orten wie zum Beispiel in Krankenhäusern oder Flugzeugen der Betrieb von Mobilfunkgeräten untersagt ist, kann ein Patient gezwungen sein, das Patientengerät zeitweilig abzuschalten.

Problematisch ist nun jedoch, dass nach dem Anschalten des Patientengerätes der Zeitpunkt des nächsten Empfangszyklus des Herzschrittmachers nicht bekannt ist und die beiden Geräte deshalb ihre Aktivitätsphasen aufeinander zeitlich abstimmen müssen. Dies kann auch geschehen, wenn der Funkkanal zwischenzeitlich von einem fremden Gerät besetzt wurde, so dass Herzschrittmacher und Patientengerät nicht miteinander kommunizieren konnten. Die genannten Beschränkungen bei der Nutzung der in Frage kommenden freigegebenen Frequenzbänder setzen jedoch enge Grenzen, da die Empfangseinheit des Herzschrittmachers auch nach verlorenem Kontakt mit dem Patientengerät nicht fortlaufend eingeschaltet sein kann.

Die der vorliegenden Erfindung zugrunde liegende Aufgabe ist deshalb, ein Verfahren zur zeitlichen Abstimmung der Aktivitätsphasen eines Senders und eines Empfängers, die jeweils nur kurzzeitig aktiv sind, einzuführen.

Das erfindungsgemäße Verfahren löst die zugrunde liegende Aufgabe, indem Sende- und Empfangseinheiten der beiden Kommunikationsmodule dergestalt an- und ausgeschaltet werden, dass die sich aus Aktivitätsphase und Aktivitätspause ergebenden Betriebsperioden für die beiden Kommunikationspartner unterschiedlich lang sind, wobei die Betriebsperiode eines der Kommunikationspartner jeweils um die Dauer einer Aktivitätsphase des anderen Kommunikationspartners länger oder Kürzer gewählt wird. Auf diese Weise verschiebt sich die relative zeitliche Lage der beiden Aktivitätsphasen der Kommunikationspartner fortlaufend gegeneinander, so dass es nach einer vorherbestimmten maximalen Zeit zu einer Überlappung der beiden Aktivitätsphasen kommen muss.

Vorteilhafterweise wird das Verfahren dergestalt durchgeführt, dass die Aktivitätsphase der Sendeeinheit des sendenden Patientengerätes gleichlang wie die Aktivitätsphase des empfangenden elektromedizinischen Implantates ist.

Ein besonders schnelles Überschneiden der Aktivitätsphasen der beiden zeitlich nicht aufeinander abgestimmten Kommunikationspartner kann erreicht werden, da die Betriebsperiode eines der Kommunikationspartner jeweils um die Dauer einer Aktivitätsphase des anderen Kommunikationspartners länger oder kürzer gewählt wird.

Um die Leistungsaufnahme der ersten Empfangseinheit des elektromedizinischen Implantates zu minimieren, ist die Dauer der Aktivitätspause der ersten Empfangseinheit bevorzugt größer als die Dauer der Aktivitätspause der ersten Sendeeinheit.

Besonders bevorzugt ist eine Variante des Verfahrens, bei dem die Dauer einer Betriebsperiode bezogen auf die Dauer der in der Betriebsperiode enthaltenen Aktivitätsphase sich nicht als ganzzahliges Vielfaches der entsprechenden Betriebsperiode des anderen Kommunikationspartners bezogen auf die Dauer dessen Aktivitätsphase ergibt. Auf diese Weise verschieben sich im Verlauf der Zeit die Aktivitätsphasen der beiden Kommunikationspartner relativ zueinander, so dass es innerhalb einer bestimmten maximalen Zeit zu deren Überlagerung kommen muss. Dies wäre nicht garantiert, wenn sich die Betriebsperioden zueinander als ganzzahlige Vielfache ergäben.

Überschneiden sich die Aktivitätsphasen der Empfangs- und Sendeeinheiten beider Kommunikationspartner, kann der empfangende Kommunikationspartner den günstigsten Zeitpunkt seiner nächsten Aktivitätsphase besonders leicht bestimmen, wenn der sendende Kommunikationspartner den Beginn seiner nächsten Aktivitätsphase als Differenz zum Zeitpunkt des aktuellen Sendevorganges überträgt. Bei einer besonders vorteilhaften Variante des erfindungsgemäßen Verfahrens sendet deshalb das Patientengerät den Zeitpunkt seiner nächsten Aktivitätsphase als Differenz zum Zeitpunkt des aktuellen Sendevorganges.

Bevorzugt schaltet das elektromedizinische Implantat nach Empfang eines den Zeitpunkt des Beginns der nächsten Aktivitätsphase der ersten Sendeeinheit des Patientengerätes übermittelnden Signals die erste Empfangseinheit bis zu dem übermittelten Zeitpunkt ab, um Energie zu sparen.

Damit die Empfangseinheit des elektromedizinischen Implantates nach einem ersten Überschneiden der Aktivitätsphasen von Sende- und Empfangseinheit möglichst nur noch dann angeschaltet wird, wenn das Patientengerät tatsächlich aktiv ist, wird in einer besonders bevorzugten Variante des Verfahrens eine Anpassung der Dauer der Aktivitätsphase und Aktivitätspause des elektromedizinischen Implantates an die Dauer der Aktivitätsphase und Aktivitätspause des Patientengerätes durchgeführt.

Ist die Kommunikationsverbindung zwischen dem elektromedizinischen Implantat und dem Patientengerät nach einer erfolgten Synchronisierung wieder zusammengebrochen, etwa weil der Sendekanal besetzt war oder das Patientengerät ausgeschaltet wurde, müssen die Aktivitätsphasen und -pausen beider Kommunikationspartner erneut aufeinander abgestimmt werden. Dazu verkürzt oder verlängert bei einer bevorzugten Variante des erfindungsgemäßen Verfahrens das elektromedizinische Implantat nach einer erfolgten Anpassung die Dauer der Aktivitätspause der ersten Empfangseinheit wieder, wenn das elektromedizinische Implantat während einer Aktivitätsphase der ersten Empfangseinheit kein Erkennungssignal vom Patientengerät empfangen hat.

Bevorzugt sendet das elektromedizinische Implantat über eine zweite Sendeeinheit ein Bestätigungssignal, sobald sich die Aktivitätsphasen beider Kommunikationsmodule überlagert haben, so dass das Patientengerät nach Empfangen des Bestätigungssignals eine periodisch fortgesetzte Kommunikationsverbindung mit dem empfangenden Kommunikationsmodul aufbauen kann.

In einer weiteren Variante des Verfahrens passt das Patientengerät nach Empfang des Bestätigungssignals die Dauer seiner Aktivitätsphase und Aktivitätspause an die des elektromedizinischen Implantates an. Auch dieses alternative Verfahren garantiert eine maximale Überlappung der Aktivitätsphasen beider Kommunikationsmodule, nach dem es zu einem ersten Überlappen der Aktivitätsphasen gekommen ist.

Wenn das Patientengerät nach einer erfolgten Synchronisierung während einer Aktivitätsphase kein Bestätigungssignal empfängt, verkürzt oder verlängert das Patientengerät bei einer bevorzugten Variante des erfindungsgemäßen Verfahrens die Dauer der Aktivitätspause der ersten Sendeeinheit, um erneut eine Synchronisierung der Aktivitätsphasen und -pausen der beiden Kommunikationspartner zu erreichen.

Die Aufgabe wird außerdem durch ein elektromedizinisches Implantat mit einer ersten Empfangseinheit, einem ersten Zeitgeber und einer ersten Kontrolleinheit gelöst, wobei die Kontrolleinheit ausgebildet ist, die erste Empfangseinheit jeweils nach vom Zeitgeber gemessenen Zeitspannen an- und auszuschalten. Die Kontrolleinheit ist bei dem erfindungsgemäßen Kommunikationsmodul dazu ausgebildet, die Zeiten, nach denen die Kontrolleinheit die Empfangseinheit an- und ausschaltet, nach Empfangen eines Erkennungssignals zu verändern und so die vom Zeitgeber bestimmten Aktivitätsphasen und -pausen an die des Patientengerätes anzupassen. Auf diese Weise kann das elektromedizinische Implantat auf einfache und effiziente Weise das Zustandekommen einer Kommunikationsverbindung erreichen.

Um die Aktivitätsphase des elektromedizinischen Implantates länger aufrechtzuerhalten, sobald sich die Aktivitätsphasen beider Kommunikationspartner erstmalig überschnitten haben, ist eine vorteilhafte Ausführung des elektromedizinischen Implantates mit einer ersten Kontrolleinheit ausgestattet, die ausgebildet ist, nach dem Empfang eines Erkennungssignals die erste Empfangseinheit erst nach dem Verstreichen einer vierten Zeitspanne abzuschalten. Dies hat den Vorteil, dass die Aktivitätsphase des elektromedizinischen Implantates nicht beendet wird, bevor die Aktivitätsphase des Patientengerätes, während derer das elektromedizinische Implantat das Erkennungssignal empfangen hat, beendet ist.

Um rechtzeitig zum Beginn der nächsten Aktivitätsphase des Patientengerätes empfangsbereit zu sein und gleichzeitig bis zu diesem Zeitpunkt keine Leistung zu verbrauchen, ist die erste Kontrolleinheit ausgebildet, nach dem Empfang eines die Dauer einer fünften Zeitspanne übermittelnden Erkennungssignales die erste Empfangseinheit nach dem Verstreichen der fünften Zeitspanne anzuschalten.

In einer Variante des elektromedizinischen Implantates verfügt dieses über eine erste Kontrolleinheit, die ausgebildet ist, nach Empfangen eines Erkennungssignals eine vorbestimmte Zeitspanne verstreichen zu lassen, bevor die erste Kontrolleinheit die Dauern der Aktivitätsphase und -pause der Empfangseinheit an die Dauer von Aktivitätsphase und -pause des Patientengerätes anpasst. Auf diese Weise kann das elektromedizinische Implantat den Beginn seiner nächsten Aktivitätsphase auf den Beginn der Aktivitätsphase des Patientengerätes legen, obgleich es nur zu einer teilweisen Überlappung der Aktivitätsphasen der beiden Kommunikationsmodule gekommen ist.

In einer weiteren Ausführung des elektromedizinischen Implantates verfügt dieses über eine Sendeeinheit, die ausgelegt ist, ein Bestätigungssignal zu senden, wenn das elektromedizinische Implantat über seine Empfangseinheit ein Erkennungssignal vom Patientengerät empfangen hat. Auf diese Weise kann das elektromedizinische Implantat dem Patientengerät signalisieren, dass es zu einer zeitlichen Abstimmung der Aktivitätsphasen der beiden Kommunikationspartner gekommen ist.

Besonders bevorzugt ist das elektromedizinische Implantat ein Herzschrittmacher oder ein Defibrillator.

Ein weiterer Aspekt betrifft ein Patientengerät mit einer ersten Sendeeinheit für das Senden in einem Sendekanal, einer zweiten Empfangseinheit und einer mit einem zweiten Zeitgeber verbundenen zweiten Kontrolleinheit. Die zweite Kontrolleinheit ist ausgebildet, die erste Sendeeinheit periodisch abwechselnd nach dem Verstreichen einer sechsten Zeitspanne abzuschalten und nach dem anschließenden Verstreichen einer siebten Zeitspanne anzuschalten. Nach dem Empfang eines Bestätigungssignals schaltet die zweite Kontrolleinheit die erste Sendeeinheit periodisch abwechselnd nach dem Verstreichen der sechsten Zeitspanne ab und nach dem anschließenden Verstreichen einer achten Zeitspanne an. Das Patientengerät ist somit ausgebildet, die Dauer seiner Aktivitätsphase und -pause an die des elektromedizinischen Implantates anzupassen, sobald es von diesem ein Bestätigungssignal empfangen hat, das eine zeitliche Überschneidung der Aktivitätsphasen beider Kommunikationspartner anzeigt.

Um andere Kommunikationsgeräte, die im selben Sendekanal senden, nicht zu stören, ist eine besonders bevorzugte Ausführung des Patientengerätes ausgebildet, den Sendekanal während einer neunten Zeitspanne, die innerhalb der Aktivitätspause der ersten Sendeeinheit liegt, auf Sendeaktivität anderer Kommunikationsgeräte zu prüfen.

Wenn der Sendekanal von anderen Kommunikationsgeräten besetzt ist, kann das Patientengerät Leistung sparen, wenn es die erste Sendeeinheit nicht anschaltet. Deshalb ist eine vorteilhafte Ausführung des Patientengerätes ausgebildet, die erste Sendeeinheit nach dem Verstreichen seiner Aktivitätspause nur dann in Betrieb zu nehmen, wenn während der neunten Zeitspanne keine Sendeaktivität anderer Kommunikationsgeräte festgestellt wurde.

Bevorzugt ist das Patientengerät ausgebildet, jeweils einmalig nach dem Verstreichen einer siebten oder achten Zeitspanne, in der das Patientengerät aufgrund von Sendeaktivität anderer Kommunikationsgeräte im Sendekanal die erste Sendeeinheit nicht angeschaltet hat, im direkten Anschluss eine zehnte Zeitspanne verstreichen und die erste Sendeeinheit während der zehnten Zeitspanne ausgeschaltet zu lassen. Die zehnte Zeitspanne beträgt dabei besonders bevorzugt die Dauer einer Aktivitätsphase und einer Aktivitätspause des elektromedizinischen Implantates, so dass die relative Verschiebung der Aktivitätsphasen der beiden Kommunikationspartner zueinander ausgesetzt wird, wenn der Sendekanal besetzt ist. Dies ist deshalb vorteilhaft, weil auf diese Weise verhindert wird, dass sich die beiden Aktivitätsphasen verfehlen, wenn während einer Überschneidung der Aktivitätsphasen der Sendekanal besetzt ist und die beiden Kommunikationspartner dadurch die Überschneidung der Aktivitätsphasen nicht feststellen konnten.

Bei einer vorteilhaften Ausführung des Patientengerätes ist die zweite Kontrolleinheit ausgebildet, nach dem Empfang eines Bestätigungssignals durch die zweite Empfangseinheit die erste Sendeeinheit erst nach dem Verstreichen einer elften Zeitspanne abzuschalten. Die elfte Zeitspanne ist dabei länger als die sechste Zeitspanne. Dies hat die Wirkung, dass das Patientengerät die Aktivitätsphase der ersten Sendeeinheit für Datenübertragung im Rahmen eine Kommunikationsverbindung mit dem elektromedizinischen Implantat verlängert, so dass mehr Daten ausgetauscht werden können.

Besonders vorteilhaft ist ein Patientengerät, das zusätzlich über eine weitere Sende- und Empfangseinheit verfügt und ausgebildet ist, über diese zusätzliche Sende- und Empfangseinheit eine drahtlose Datenverbindung aufzubauen und die von der ersten Empfangseinheit empfangenen Daten über diese Sendeeinheit zu senden.

Die Erfindung soll nun anhand eines Ausführungsbeispiels mit Bezug auf die Zeichnungen näher erläutert werden. Diese zeigen in:
- Abbildung 1:: ein Flussdiagramm eines Beispiels des erfindungsgemäßen Verfahrens;
- Abbildung 2:: ein Blockdiagramm eines Systems, welches ein elektromedizinisches Implantat und ein Patientengerät umfasst.

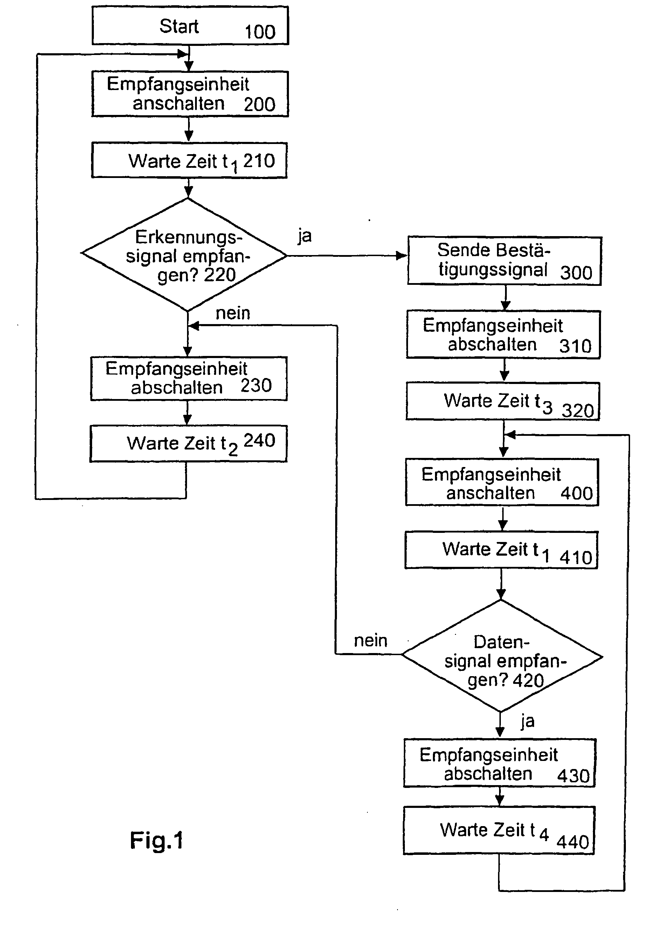

Eine beispielhafte Variante des erfindungsgemäßen Verfahrens zum Herstellen einer drahtlosen Kommunikationsverbindung zwischen einem Patientengerät und einem elektromedizinischen Implantat wird in Abbildung 1 gezeigt. Es beginnt nach dem Anschalten des elektromedizinischen Implantates mit dem Start 100. Anschließend werden zyklisch die Schritte 200 bis 240 durchlaufen, bis eine Verzweigungsbedingung 220 erfüllt ist.

Im Schritt 200 wird die Empfangseinheit des elektromedizinischen Implantates angeschaltet, woraufhin das elektromedizinische Implantat eine vorherbestimmte Zeit t₁ (Schritt 210) wartet. Während der Zeitspanne t₁, der Aktivitätsphase der Empfangseinheit, prüft das elektromedizinische Implantat, ob über die eingeschaltete Empfangseinheit ein Erkennungssignal des Patientengerätes empfangen wurde. Nach dem Verstreichen der Zeit t₁ wird im Schritt 220 zum Schritt 230 verzweigt, wenn innerhalb der Zeit t₁ kein Erkennungssignal empfangen wurde. Andernfalls wird zum Schritt 300 verzweigt.

Ist kein Erkennungssignal empfangen worden, wird in Schritt 230 die Empfangseinheit des elektromedizinischen Implantates wieder abgeschaltet. Anschließend wird eine zweite vorherbestimmt Zeit t₂, die Aktivitätspause, abgewartet (Schritt 240), bevor zum Schritt 200 zurückgesprungen wird.

Wurde im Schritt 220 festgestellt, dass während der Zeit t₁ das Erkennungssignal vom Patientengerätes empfangen wurde, dann wird in Schritt 300 ein Bestätigungssignal an das Patientengerät gesandt, woraufhin im Schritt 310 die Empfangseinheit abgeschaltet wird. Anschließend wird in Schritt 320 eine Zeit t₃ verstreichen gelassen, die so bestimmt ist, dass das Ende dieser Zeit mit dem zu erwartenden Beginn der nächsten Aktivitätsphase der Sendeeinheit des Patientengerätes zusammenfällt. Die Zeit t₃ kann beispielsweise zuvor durch das Patientengerät als Teil des Erkennungssignal an das elektromedizinische Implantat übermittelt worden sein.

Im Anschluss an den Schritt 320 werden die Schritte 400 bis 440 zyklische wiederholt. In Schritt 400 wird wiederum die Empfangseinheit des elektromedizinischen Implantates angeschaltet und während der Zeit t₁ das Senden und Empfangen von Daten zwischen dem elektromedizinischen Implantat und dem Patientengerät wie gewohnt vorgenommen (Schritt 410). In Schritt 420 prüft das elektromedizinische Implantat, ob es während der Zeitspanne t₁ Daten oder ein Erkennungssignal vom Patientengerät empfangen hat. Ist dies nicht der Fall, dann ist die Synchronisation der Aktivitätsphasen und -pausen der beiden Kommunikationspartner verloren gegangen und es muss in den Suchmodus der Schritte 200 bis 240 zurückgesprungen werden. Konnte der Datenaustausch jedoch ohne Beeinträchtigung durchgeführt werden, wird in Schritt 430 die Empfangseinheit des elektromedizinischen Implantates abgeschaltet. In Schritt 440 wird eine Zeit t₄ verstreichen gelassen, die der Dauer der Aktivitätspause des Patientengerätes entspricht. In dem die Länge der Aktivitätspause des einen Kommunikationspartners an die des anderen Kommunikationspartners angepasst wird, wird eine maximale Überschneidung der Aktivitätsphasen beider Kommunikationspartner im nächsten Sendezyklus sichergestellt.

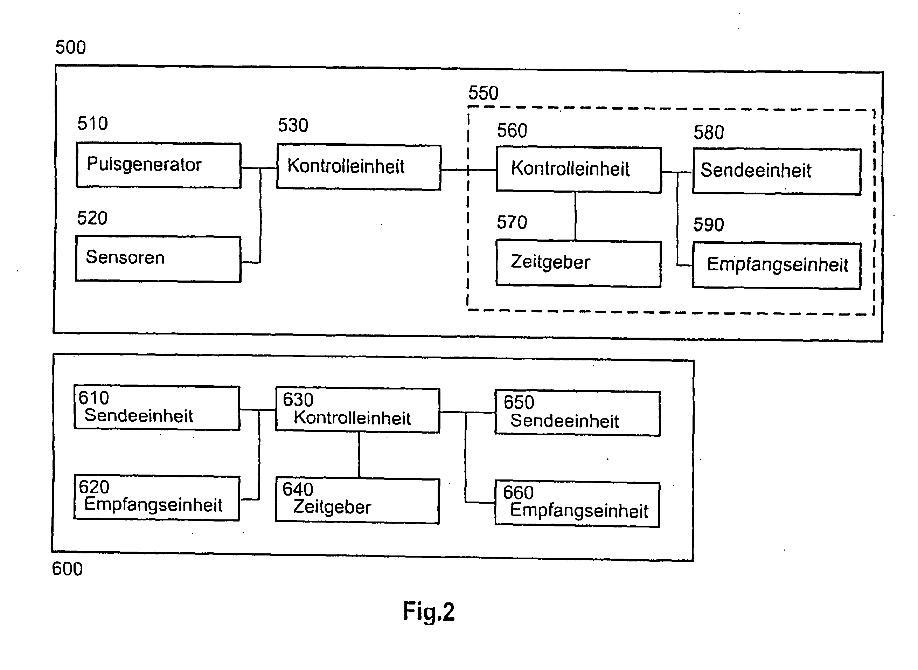

Die Abbildung 2 zeigt ein Blockdiagramm eines Kommunikationssystems mit einem elektromedizinischen Implantat 500 auf der einen Seite und einem Patientengerät 600 auf der anderen Seite.

Das elektromedizinische Implantat 500 gliedert sich in solche Blöcke, die medizinische Funktionen ausführen, und ein Kommunikationsmodul 550. Das Kommunikationsmodul 550 ist auf der rechten Seite des elektromedizinischen Implantates 500 zu sehen und gliedert sich in vier Unterblöcke. Diese Unterblöcke umfassen die erste Kontrolleinheit 560 und den mit ihr verbundenen ersten Zeitgeber 570 sowie die zweite Sendeeinheit 580 und die erste Empfangseinheit 590. Als medizinische Funktionsblöcke wurden ein Pulsgenerator 510, ein medizinische Sensoren enthaltener Block 520 und eine dritte Kontrolleinheit 530 beispielhaft aufgenommen. Diese Funktionsblöcke könnten die wesentlichen Funktionen eines Herzschrittmachers verwirklichen. Die dritte Kontrolleinheit 530 würde in einem solchen Herzschrittmacher über die Sensoren 520 beispielsweise die Herzschlagrate kontrollieren und, sollte diese unter einen vorgegebenen Wert fallen, den Pulsgenerator 510 veranlassen, Spannungspulse zur Stimulation des Herzens abzugeben.

Die Blöcke 560 bis 590 des Kommunikationsmoduls 550 sind geeignet, das erfindungsgemäße Verfahren durchzuführen. Der erste Zeitgeber 570 bestimmt dabei die Zeiten t₁ bis t₄, die zum Beispiel in den Schritten 210, 240, 320 und 440 verstreichen gelassen werden. Die Zeit t₁ kann dabei der ersten Zeitspanne, t₂ der zweiten Zeitspanne, t₃ der fünften Zeitspanne und t₄ der dritten Zeitspanne entsprechen. Die erste Kontrolleinheit 560 schaltet die zweite Sendeeinheit 580 und die erste Empfangseinheit 590 nach vom ersten Zeitgeber 570 bestimmten Zeiten an oder aus. Indem sie das tut, senkt sie die Leistungsaufnahme der zweiten Sendeeinheit 580 und der ersten Empfangseinheiten 590, so dass ein elektromedizinisches Implantat, das mit einem solchen Kommunikationsmodul ausgestattet ist, mit derselben Batterieleistung länger im Körper eines Patienten verbleiben kann.

Ein System umfasst ein elektromedizinisches Implantat 500 und ein Patientengerät 600. Die ersten vier Blöcke des Patientengerätes 600 sind, analog zu den vier Blöcken des Kommunikationsmoduls 550 des elektromedizinischen Implantats 500, die erste Sendeeinheit 610, die zweite Empfangseinheit 620, die zweite Kontrolleinheit 630 und der mit ihr verbundene zweite Zeitgeber 640. Weiterhin verfügt das Patientengerät wenigstens noch über eine weitere, dritte Sendeeinheit 650 und eine dritte Empfangseinheit 660. Die zweite Kontrolleinheit 630 schaltet die erste Sendeeinheit 610 und die zweite Empfangseinheit 620 nach vom zweiten Zeitgeber 640 bestimmten Zeiten t₁ und t₄ an oder aus. Sind die beiden Kommunikationspartner 500 und 600 anfänglich nicht miteinander synchronisiert, so dass sich die Aktivitätsphasen beider Geräte, während derer die Sende- und Empfangseinheiten 580, 590, 610 und 620 angeschaltet sind, nicht überlappen, wird durch das zuvor beschriebene, erfindungsgemäße Verfahren wegen der unterschiedlich langen Aktivitätspausen t₂ und t₄ der beiden Kommunikationspartner eine Überschneidung der Aktivitätsphasen innerhalb einer begrenzten Zeitspanne sichergestellt. Empfängt das Patientengerät 600 über seine erste Empfangseinheit 620 Daten vom elektromedizinischen Implantat 500, leitet es diese über die dritte Sendeeinheit 650 sofort oder beim nächsten Zustandekommen einer Kommunikationsverbindung mit dem Home Monitoring Service Center an dieses weiter. Die dritte Empfangseinheit 660 wird zum Aufbau einer drahtlosen Kommunikationsverbindung zum Home Monitoring Service Center benötigt, kann in einer Variante des erfindungsgemäßen Patientengerätes aber auch dazu dienen, Daten vom Home Monitoring Service Center entgegenzunehmen. Diese Daten können die Funktion des Patientengerätes 600 bestimmende Betriebsparameter und -software oder Betriebsparameter für das elektromedizinische Implantat 500 sein, die vom Patientengerät 600 über die erste Sendeeinheit 610 an das elektromedizinische Implantat 500 weitergeleitet werden.

Das gewöhnlich batteriebetriebene Patientengerät 600 erreicht ebenfalls durch das fortlaufende Ein- und Abschalten seiner Sende- und Empfangseinheiten 610, 620, 650 und 660 eine längere Laufzeit, bevor seine Batterien vom Patienten aufgeladen werden müssen. Indem es mittelbar die Kommunikation zwischen dem elektromedizinischen Implantat 500 und dem Home Monitoring Service Center ermöglicht, senkt es den Leistungsbedarf des elektromedizinischen Implantates 500 erheblich.

## Patentansprüche

1. Verfahren zum Herstellen einer drahtlosen Kommunikationsverbindung zwischen einem Patientengerät (600) und einem elektromedizinischen Implantat (500) als Kommunikationspartnern,
bei dem eine erste Sendeeinheit (610) des Patientengerätes (600) fortlaufend ein- und ausgeschaltet wird, so dass sich die erste Sendeeinheit (610) abwechselnd in einer Aktivitätsphase und einer Aktivitätspause befindet, und während ihrer Aktivitätsphase mindestens einmal ein Erkennungssignal sendet,
und bei dem eine erste Empfangseinheit (590) des elektromedizinischen Implantates (500) fortlaufend ein- und ausgeschaltet wird, so dass sich die erste Empfangseinheit (590) abwechselnd in einer Aktivitätsphase und einer Aktivitätspause befindet, und während ihrer Aktivitätsphase prüft, ob sich die erste Sendeeinheit (610) gerade in ihrer Aktivitätsphase befindet und ein Erkennungssignal sendet,
wobei die Dauern der Aktivitätsphasen kürzer als die Dauern der Aktivitätspausen der ersten Sende- bzw. Empfangseinheit (610, 590) des jeweils anderen Kommunikationspartners sind,
und wobei das Ein- und Ausschalten der ersten Empfangseinheit (590) so durchgeführt wird, dass eine Aktivitätsphase und eine Aktivitätspause der ersten Empfangseinheit (590) zusammen eine Gesamtdauer ergeben, die von der Gesamtdauer einer Aktivitätsphase und einer Aktivitätspause der ersten Sendeeinheit (610) abweicht, so dass sich innerhalb einer absehbaren Zeitspanne die Aktivitätsphasen der ersten Sendeeinheit (610) und der ersten Empfangseinheit (590) überschneiden und eine drahtlose Kommunikationsverbindung zwischen den beiden Kommunikationspartnern (600, 500) zustande kommt,
**dadurch gekennzeichnet,**
**dass** das Ein- und Ausschalten der ersten Sendeeinheit (610) und der ersten Empfangseinheit (590) dergestalt erfolgen, dass sich die Gesamtdauer einer Aktivitätsphase und einer Aktivitätspause der ersten Sendeeinheit (610) von der Gesamtdauer einer Aktivitätsphase und einer Aktivitätspause der ersten Empfangseinheit (590) um die Dauer einer Aktivitätsphase der ersten Empfangseinheit (590) oder der ersten Sendeeinheit (610) unterscheidet.

2. Verfahren nach Anspruch 1, **dadurch gekennzeichnet,**
**dass** das Ein- und Ausschalten der ersten Sendeeinheit (610) und der ersten Empfangseinheit (590) dergestalt erfolgen, dass die jeweilige Dauer einer Aktivitätsphase der ersten Sendeeinheit (610) und einer Aktivitätsphase der ersten Empfangseinheit (590) gleich lang sind.

3. Verfahren nach einem der vorhergehenden Ansprüche, **dadurch gekennzeichnet,**
**dass** die Dauer der Aktivitätspause der ersten Empfangseinheit (590) größer ist als die Dauer der Aktivitätspause der ersten Sendeeinheit (610).

4. Verfahren nach einem der vorhergehenden Ansprüche, **dadurch gekennzeichnet,**
**dass** das Ein- und Ausschalten der ersten Sendeeinheit (610) und der ersten Empfangseinheit (590) dergestalt erfolgen, dass sich der Quotient aus der jeweiligen Gesamtdauer einer Aktivitätspause und einer Aktivitätsphase geteilt durch die Dauer einer Aktivitätsphase für den einen Kommunikationspartner (500 oder 600) sich nicht als ganzzahliges Vielfaches des entsprechenden Quotienten des anderen Kommunikationspartners (500 oder 600) errechnet.

5. Verfahren nach einem der vorhergehenden Ansprüche, **dadurch gekennzeichnet,**
**dass** das Patientengerät (600) während der Aktivitätsphase der ersten Sendeeinheit (610) den Zeitpunkt des Beginns der nächsten Aktivitätsphase der ersten Sendeeinheit (610) sendet, wobei dieser Zeitpunkt als Differenz zum Zeitpunkt des Sendens angegeben wird.

6. Verfahren nach Anspruch 5, **dadurch gekennzeichnet,**
**dass** das elektromedizinische Implantat (600) nach dem Empfang eines den Zeitpunkt des Beginns der nächsten Aktivitätsphase der ersten Sendeeinheit (610) übermittelnden Signals die erste Empfangseinheit (590) abschaltet und zu dem übermittelten Zeitpunkt des Beginns der nächsten Aktivitätsphase der ersten Sendeeinheit (610) die erste Empfangseinheit (590) wieder anschaltet.

7. Verfahren nach einem der vorhergehenden Ansprüche, **dadurch gekennzeichnet,**
**dass** das elektromedizinische Implantat (500) die Dauer von Aktivitätsphase und -pause der ersten Empfangseinheit (590) an die Dauer von Aktivitätsphase und -pause der ersten Sendeeinheit (610) im Falle der zeitlichen Überlagerung der Aktivitätsphasen der ersten Sendeeinheit (610) und der ersten Empfangseinheit (590) anpasst.

8. Verfahren nach Anspruch 7, **dadurch gekennzeichnet,**
**dass** das elektromedizinische Implantat die Dauer der Aktivitätspause der ersten Empfangseinheit (590) nach einer erfolgten Anpassung an die Dauer von Aktivitätsphase und -pause der ersten Sendeeinheit (610) verkürzt oder verlängert, wenn während einer Aktivitätsphase der ersten Empfangseinheit (590) kein von dem Patientengerät (600) gesandtes Erkennungssignal empfangen wurde.

9. Verfahren nach einem der Ansprüche 1 bis 6, **dadurch gekennzeichnet,**
**dass** das elektromedizinische Implantat (500) über eine zweite Sendeeinheit (580) ein Bestätigungssignal sendet, wenn sich die Aktivitätsphasen der ersten Sendeeinheit (610) und der ersten Empfangseinheit (590) überlagert haben und die erste Empfangseinheit (590) das Erkennungssignal der ersten Sendeeinheit (610) empfangen hat.

10. Verfahren nach Anspruch 8, **dadurch gekennzeichnet,**
**dass** das Patientengerät (600) die Dauer von Aktivitätsphase und -pause der ersten Sendeeinheit (610) an die Dauer von Aktivitätsphase und - pause der ersten Empfangseinheit (590) im Falle des Empfangs eines von dem elektromedizinischen Implantat (500) gesandten Bestätigungssignals über eine zweite Empfangseinheit (620) anpasst.

11. Verfahren nach Anspruch 9, **dadurch gekennzeichnet,**
**dass** das Patientengerät die Dauer der Aktivitätspause der ersten Sendeeinheit (610) nach einer erfolgten Anpassung an die Dauer von Aktivitätsphase und -pause der ersten Empfangseinheit (590) verkürzt oder verlängert, wenn während einer Aktivitätsphase der ersten Sendeeinheit (610) kein von dem elektromedizinischen Implantat (500) gesandtes Bestätigungssignal über die zweite Empfangseinheit (620) empfangen wurde.

## Claims

1. A method for establishing a wireless communication connection between a patient device (600) and an electromedical implant (500), which function as communication partners,
in which a first transmission unit (610) of the patient device (600) is switched on and off continuously, and so the first transmission unit (610) alternates between an activity phase and an activity pause, and transmits an identification signal at least once during its activity phase,
and in which a first receiving unit (590) of the electromedical implant (500) is switched on and off continuously, and so the first receiving unit (590) alternates between an activity phase and an activity pause, and, during its activity phase, checks to determine whether the first transmission unit (610) is in its activity phase at that moment and transmits an identification signal,
wherein the durations of the activity phases are shorter than the durations of the activity pauses of the first transmission unit or receiving unit (610, 590) of the other communication partner,
and wherein the first receiving unit (590) is switched on and off such that the combination of an activity phase and an activity pause of the first receiving unit (590) results in a total duration that deviates from the total duration of an activity phase and an activity pause of the first transmission unit (610), and therefore the activity phases of the first transmission unit (610) and of the first receiving unit (590) overlap within a foreseeable period, and a wireless communication connection is established between the two communication partners (600, 500), **characterized in that** the first transmission unit (610) and the first receiving unit (590) are switched on and off such that the total duration of an activity phase and an activity pause of the first transmission unit (610) differs from the total duration of an activity phase and an activity pause of the first receiving unit (590) by the duration of an activity phase of the first receiving unit (590) or of the first transmission unit (610).

2. The method according to claim 1, **characterized in that** the first transmission unit (610) and the first receiving unit (590) are switched on and off such that the duration of an activity phase of the first transmission unit (610) and the duration of an activity phase of the first receiving unit (590) are equal.

3. The method according to one of the preceding claims, **characterized in that** the duration of the activity pause of the first receiving unit (590) is longer than the duration of the activity pause of the first transmission unit (610).

4. The method according to one of the preceding claims, **characterized in that** the first transmission unit (610) and the first receiving unit (590) are switched on and off such that the quotient of the particular total duration of an activity pause and an activity phase, divided by the duration of an activity phase for one of the communication partners (500 or 600), is not a whole-number multiple of the corresponding quotient of the other communication partner (500 or 600).

5. The method according to one of the preceding claims, **characterized in that**, during the activity phase of the first transmission unit (610), the patient device (600) transmits the time at which the next activity phase of the first transmission unit (610) begins, this time being indicated as the difference from the time at which transmission took place.

6. The method according to claim 5, **characterized in that**, after receiving a signal that communicates the time at which the next activity phase of the first transmission unit (610) begins, the electromedical implant (600) switches off the first receiving unit (590), and it switches the first receiving unit (590) back on at the time that was communicated as being the start of the next activity phase of the first transmission unit (610).

7. The method according to one of the preceding claims, **characterized in that** the electromedical implant (500) adapts the duration of the activity phase and the activity pause of the first receiving unit (590) to the duration of the activity phase and the activity pause of the first transmission unit (610) if the activity phases of the first transmission unit (610) and of the first receiving unit (590) overlap in terms of time.

8. The method according to claim 7, **characterized in that**, after an adaptation to the duration of the activity phase and the activity pause of the first transmission unit (610), the electromedical implant shortens or extends the duration of the activity pause of the first receiving unit (590) if an identification signal transmitted by the patient device (600) was not received during an activity phase of the first receiving unit (590).

9. The method according to one of the claims 1 through 6, **characterized in that** the electromedical implant (500) transmits a confirmation signal via a second transmission unit (580) if the activity phases of the first transmission unit (610) and the first receiving unit (590) have overlapped and the first receiving unit (590) has received the identification signal from the first transmission unit (610).

10. The method according to claim 8, **characterized in that** the patient device (600) adapts the duration of the activity phase and the activity pause of the first transmission unit (610) to the duration of the activity phase and the activity pause of the first receiving unit (590) if a confirmation signal transmitted by the electromedical implant (500) is received via a second receiving unit (620).

11. The method according to claim 9, **characterized in that**, after an adaptation to the duration of the activity phase and the activity pause of the first receiving unit (590), the patient device shortens or extends the duration of the activity pause of the first transmission unit (610) if a confirmation signal transmitted by the electromedical implant (500) was not received via the second receiving unit (620) during an activity phase of the first transmission unit (610).

## Revendications

1. Méthode pour établir une connexion de communication sans fil, entre un dispositif patient (600) et un implant électro-médical (500) en tant que partenaires de communication,
dans laquelle une première unité de transmission (610) du dispositif patient (600) est allumée et éteinte continuellement, et la première unité de transmission (610) alterne ainsi entre une phase d'activité et une pause d'activité, et transmet un signal de détection, au moins une fois pendant sa phase d'activité,
et dans laquelle une première unité de réception (590) de l'implant électro-médical (500) est allumée et éteinte continuellement, et la première unité de réception (590) alterne donc entre une phase d'activité et une pause d'activité et vérifie, pendant sa phase d'activité, si la première unité de transmission (610) est à cet instant dans sa phase d'activité, et transmet un signal de détection,
où les durées des phases d'activité sont plus courtes que les durées des pauses d'activité de la première unité de transmission ou unité de réception (610, 590) de l'autre partenaire de communication,
et où la première unité de réception (590) est allumée et éteinte, de sorte que la combinaison d'une phase d'activité et d'une pause d'activité de la première unité de réception (590) résulte dans une durée totale déviant de la durée totale d'une phase d'activité et d'une pause d'activité de la première unité de transmission (610), et par conséquent, les phases d'activité de la première unité de transmission (610) et de la première unité de réception (590) se chevauchent en l'espace d'une période prévisible, et une connexion de communication sans fil est établie entre les deux partenaires de communication (600, 500),
**caractérisée en ce que**
la première unité de transmission (610) et la première unité de réception (590) sont allumées et éteintes, de sorte que la durée totale d'une phase d'activité et d'une pause d'activité de la première unité de transmission (610) diffère de la durée totale d'une phase d'activité et d'une pause d'activité de la première unité de réception (590), pour l'équivalent de la durée d'une phase d'activité de la première unité de réception (590) et de la première unité de transmission (610).

2. Méthode selon la revendication 1, **caractérisée en ce que** la première unité de transmission (610) et la première unité de réception (590) sont allumées et éteintes, de telle manière que la durée d'une phase d'activité de la première unité de transmission (610) et la durée d'une phase d'activité de la première unité de réception (590) sont identiques.

3. Méthode selon l'une des revendications précédentes, **caractérisée en ce que** la durée d'une pause d'activité de la première unité de réception (590) est supérieure à la durée d'une pause d'activité de la première unité de transmission (610).

4. Méthode selon l'une des revendications précédentes, **caractérisée en ce que** la première unité de transmission (610) et la première unité de réception (590) sont allumées et éteintes de telle manière que le quotient de la durée totale particulière d'une pause d'activité et d'une phase d'activité, divisée par la durée d'une phase d'activité pour l'un des partenaires de communication (500 ou 600), n'est pas un multiple entier du quotient correspondant de l'autre partenaire de communication (500 ou 600).

5. Méthode selon l'une des revendications précédentes, **caractérisée en ce que** pendant la phase d'activité de la première unité de transmission (610), le dispositif patient (600) transmet le moment où commence la prochaine phase d'activité de la première unité de transmission (610), ce moment étant indiqué comme la différence par rapport au moment où la transmission a eu lieu.

6. Méthode selon la revendication 5, **caractérisée en ce que** après la réception d'un signal communiquant le moment où commence la prochaine phase d'activité de la première unité de transmission (610), l'implant électro-médical (600) éteint la première unité de réception (590), et rallume la première unité de réception (590) au moment qui a été communiqué comme étant le début de la prochaine phase d'activité de la première unité de transmission (610).

7. Méthode selon l'une des revendications précédentes, **caractérisée en ce que** l'implant électro-médical (500) adapte la durée de la phase d'activité et de la pause d'activité de la première unité de réception (590) à la durée de la phase d'activité et de la pause d'activité de la première unité de transmission (610) si les phases d'activité de la première unité de transmission (610) et de la première unité de réception (590) se chevauchent en terme de temps.

8. Méthode selon la revendication 7, **caractérisée en ce que** après l'adaptation de la durée de la phase d'activité et de la pause d'activité de la première unité de transmission (610), l'implant électro-médical raccourcit ou rallonge la durée de la pause d'activité de la première unité de réception (590) si un signal de détection transmis par le dispositif patient (600) n'a pas été reçu pendant une phase d'activité de la première unité de réception (590).

9. Méthode selon l'une des revendications 1 à 6, **caractérisée en ce que** l'implant électro-médical (500) transmet un signal de confirmation par le biais d'une deuxième unité de transmission (580), si les phases d'activité de la première unité de transmission (610) et de la première unité de réception (590) se sont chevauchées et si la première unité de réception (590) a reçu le signal de détection de la première unité de transmission (610).

10. Méthode selon la revendication 8, **caractérisée en ce que** le dispositif patient (600) adapte la durée de la phase d'activité et de la pause d'activité de la première unité de transmission (610) à la durée de la phase d'activité et de la pause d'activité de la première unité de réception (590) si un signal de confirmation transmis par l'implant électro-médical (500) est reçu par une deuxième unité de réception (620).

11. Méthode selon la revendication 9, **caractérisée en ce que** après une adaptation à la durée de la phase d'activité et de la pause d'activité de la première unité de réception (590), le dispositif patient raccourcit ou rallonge la durée de la pause d'activité de la première unité de transmission (610), si un signal de confirmation transmis par l'implant électro-médical (500) n'a pas été reçu par la deuxième unité de réception (620) pendant une phase d'activité de la première unité de transmission (610).
